# EUROPEAN PATENT SPECIFICATION

(11) **EP 2 855 043 B1**
(45) Date of publication and mention of the grant of the patent: **03.05.2023**
(21) Application number: 13793791.8
(22) Date of filing: 23.05.2013
(51) Int. Cl.: A61B 17/06, B21F 1/06, B21F 35/02, B21F 15/02, B21D 37/10

(54) **SYSTEM AND METHOD FOR MAKING TAPERED LOOPED SUTURE**
SYSTEM UND VERFAHREN ZUR HERSTELLUNG EINER VERJÜNGTEN SCHLAUFENNAHT
SYSTÈME ET PROCÉDÉ DE RÉALISATION D'UNE SUTURE À BOUCLE EFFILÉE

(30) Priority: 25.05.2012 US 201213480614
(43) Date of publication of application: 08.04.2015
(73) Proprietor: Covidien LP, Mansfield, MA 02048 (US)
(72) Inventor: MAIORINO, Nicholas, Branford, Connecticut 06405 (US); KOSA, Timothy, Hamden, Connecticut 06514 (US); BUCHTER, Mark, Ansonia, Connecticut 06401 (US); KROEBER, Keith, Portland, Connecticut 06480 (US); HART, Richard Casey, Clinton, Connecticut 06413 (US); SUSZYNSKI, Gary, Woodbury, Connecticut 06798 (US)
(74) Representative: Soames, Candida Jane
(86) International application number: PCT/US2013/042340
(87) International publication number: WO 2013/177351

(56) References cited:
- WO-A1-96/25109
- US-A- 4 575 372
- US-A- 6 089 438
- US-A1- 2002 029 065
- US-A1- 2002 035 371
- US-A1- 2009 216 269
- US-A1- 2010 101 707
- US-A1- 2011 180 196
- US-A1- 2012 024 482

## Description

### BACKGROUND

### Technical Field

The present disclosure relates to a system of forming a looped suture. More particularly, the present disclosure relates to an automated system of forming a looped suture having a tapered cut.

### Background of Related Art

The forming of a loop in a suture is known, as are methods of forming the loop. A loop may be formed in a suture for a number of reasons. For example, during manufacture a loop may be formed in the suture to assist in further processing of the suture, e.g., for holding the suture as barbs are formed along the length thereof. Alternatively, a loop formed in a suture during manufacture may be used to secure the suture to tissue. In this manner, once the non-looped end of the suture is inserted through tissue, that end may be threaded through the loop to form a slip knot-like configuration that may be tied to secure tissue. In another application, a loop may be formed in a suture in place of a knot. This requires the use of a handheld instrument that may be brought into an operating room.

Therefore, it would be beneficial to have a system and method of forming a looped suture to include a taper cut.

US2011180196 discloses an active anvil assembly of the type on which the preamble to the independent claim is based.

### SUMMARY

According to the invention there is provided an active anvil assembly for use in forming a loop suture as recited in independent claim 1 with preferred features being set forth in the dependent claims. Further aspects of the invention provide a system and a method for forming a looped suture.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
FIG. 1A is a side view of a looped suture including a tapered portion;
FIG. 1B is a cross-sectional end view of the looped suture of FIG. 1B, taken along line 1B-1B;
FIG. 1C is an enlarged side view of FIG. 1A;
FIG. 2 is a side view of a tapered loop forming system of the present disclosure;
FIG. 3 is an enlarged side view of a suture supply assembly of the tapered loop forming system of FIG. 2;
FIG. 4 is an enlarged sectional side view of the tapered loop forming system of FIG. 2;
FIG. 5 is an enlarged side view of carriage assembly of the tapered loop forming system of FIG. 2;
FIG. 6A is an enlarged side view of embodiments of portions of the welding assembly and the trimming assembly of FIG. 2;
FIG. 6B is a perspective view of portions of the active anvil assembly and trimming assembly of FIG. 4;
FIG. 6C is a perspective view of portions of the active anvil assembly of FIGS. 6A and 6B;
FIG. 7A is an enlarged cross-sectional side view of welding assembly of the loop forming system of FIG. 2, in a partially activated position; and
FIG. 7B is an enlarged cross-sectional side view of the welding assembly of FIG. 7A, in a fully activated position.

### DETAILED DESCRIPTION

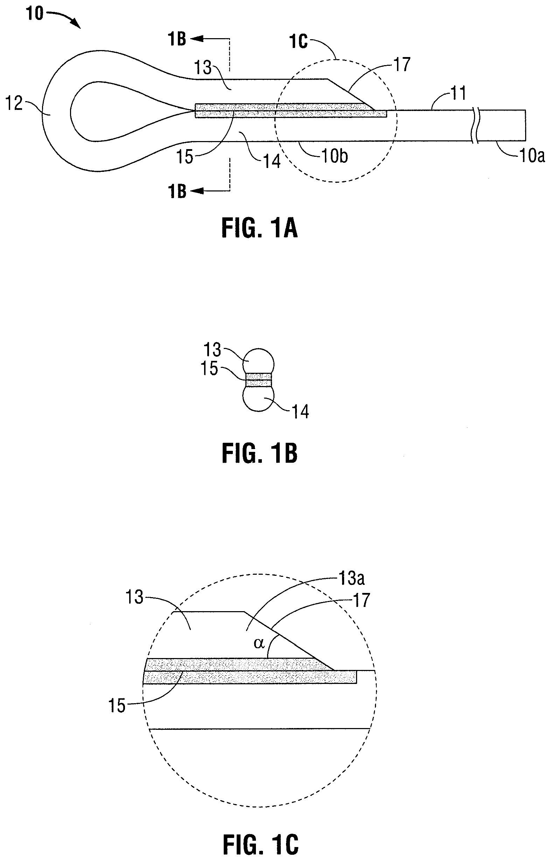

A system and method for forming a looped suture including a tapered cut is described herein. Referring initially to FIG. 1A, a looped suture formed in accordance with the method of the present disclosure is shown generally as looped suture 10. Suture 10 is formed from a monofilament thread 11, however, it is envisioned that suture 10 may include braided threads, multifilament threads and other surgical fibers. Although shown having a circular cross-sectional geometry, the cross-sectional geometry of thread 11 may be of any suitable shape. For example, thread 11 may be round, elliptical, square, flat, triangular, octagonal, and rectangular. Thread 11 may be formed of degradable materials, non-degradable materials, and combinations thereof. Thread 11 may be formed using any technique within the purview of those skilled in the art, such as, for example, extrusion, molding and/or solvent casting.

With reference to FIGS. 1A-1C, looped suture 10 includes a loop 12 formed on a distal end 10b of the suture 10. Loop 12 forms a substantially teardrop shape and may be formed of any size. Although a substantially teardrop-shaped loop 12 is illustrated, other variations, such as circular, oval and spherical-shaped loops are envisioned. A first section 13 of monofilament thread 11 overlays a second section 14 of thread 11 to form loop 12. The adjacent surfaces of first and second sections 13, 14 form a joined segment or joint 15. As shown, joined segment 15 extends beyond first section 13 of thread 11. In this manner, first and second sections 13, 14 of thread 11 are less likely to separate or peel away from each other as looped suture 10 is pulled through tissue (not shown).

As will be described in further detail below, first and second sections 13, 14 of thread 11 are welded together to form joined section 15. In this manner, first and second sections 13, 14 of thread 11 are locally heated until each fuses to form joined segment 15. Various types of energy may be used to locally heat first and second sections 13, 14 to form joined segment 15, including, radio frequency (RF), ultrasonic, laser, electrical arc discharge, and thermal. Alternatively, first and second sections 13, 14 of thread 11 may be joined using glue, epoxy or other adhesive.

With particular reference to FIG. 1C, a proximal end 13a of first section 13 is angled to form a tapered surface 17. Tapered surface 17 angles downwardly towards proximal end 10a (FIG. 1A) of looped suture 10. Tapered surface 17 may form an angle between zero degrees (0°) and ninety degrees (90°), and preferably between about fifteen degrees (15°) to about sixty degrees (60°). Tapered surface 17 facilitates insertion of loop 12 into or through tissue. Tapered surface 17 may be formed prior to, during or following the joining of first and second sections 13, 14.

Although shown having a substantially planar taper, tapered surface 17 may include any number of configurations. For example, tapered surface 17 may be beveled, may include a laterally and longitudinally concave taper, may include a laterally and longitudinally convex taper, or may include any combination thereof. Tapered surface 17 may be selected depending on the tissue being sutured and/or the depth loop 12 is desired to be received within the tissue.

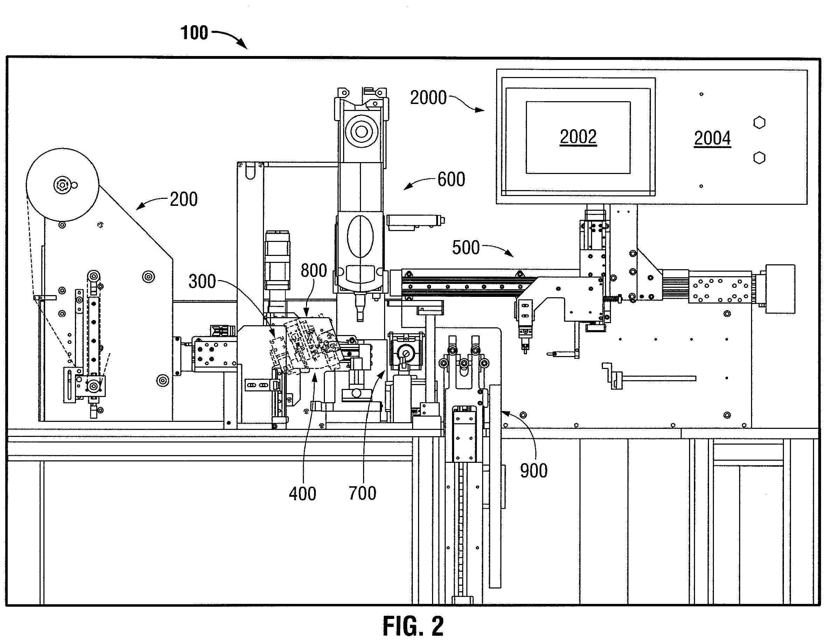

A system for forming loop 12 on distal end 10b of looped suture 10 will now be described with reference to FIGS. 2-7B, and is shown generally as tapered loop forming system 100. Although shown as being automated, it is envisioned that various components and/or process within tapered loop forming system 100 may manually completed. Referring initially to FIG. 2, system 100 includes a suture supply assembly 200, an initial gripping assembly 300, a flipper gripping assembly 400, a carriage assembly 500, a welding assembly 600, a trimming assembly 700, a cutter assembly 800, and may optionally include a thread lengthening assembly 900 and a monitoring assembly 2000.

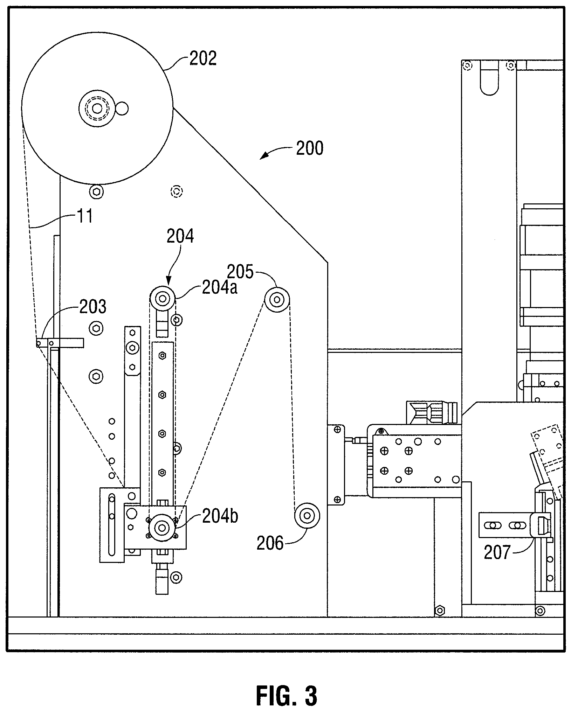

With reference now to FIG. 3, supply assembly 200 is configured to provide thread 11 to initial gripping assembly 300. Supply assembly 200 includes a spool 202, a first guide member 203, a roller assembly 204, first and second rollers 205, 206 and a second guide member 207. First guide member 203 is configured to direct thread 11 from spool 202 to roller assembly 204. Roller assembly 204 includes a set of fixed rollers 204a and a set of adjustable rollers 204b. Roller assembly 204 is configured to receive thread 11 about fixed and adjustable rollers 204a, 204b a plurality of times. As shown, rollers 204a, 204b are configured to receive thread 11 thereabout four (4) times, however, roller assembly 204 may be configured to receive thread 11 thereabout more or less than four times. First and second rollers 205, 206 are positioned to direct thread 11 through second guide member 207. Although shown including supply assembly 200 for providing a continuous supply of thread 11 from spool 202, alternative supply assemblies are known and may be modified for use with system 100. For example, thread 11 may be provided in fixed or predetermined lengths rather than continuously from a spool. In this manner, the aspects of the present disclosure should not be read as limited to the supply assembly herein disclosed.

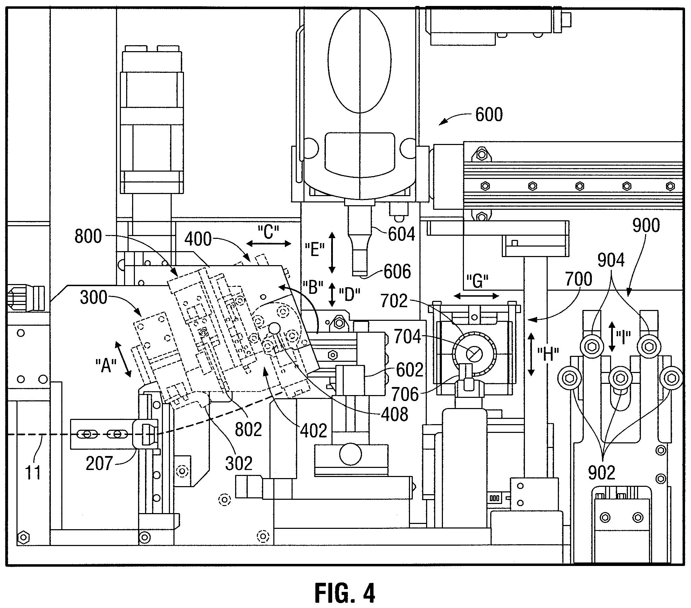

Turning now to FIG. 4, initial gripping assembly 300 includes an initial gripper 302 configured to selectively engage and selectively grasp thread 11 throughout the loop end forming process. Initial gripping assembly 300 translates on a diagonal, in the direction of arrows "A". During the looped end forming process, initial gripping assembly 300 is activated to grasp a proximal end of thread 11 when tension is applied to a distal end of thread 11 to prevent excess thread from being pulled from supply assembly 200. In this manner, initial gripping assembly 300 may include any device or apparatus capable of selectively grasping thread 11.

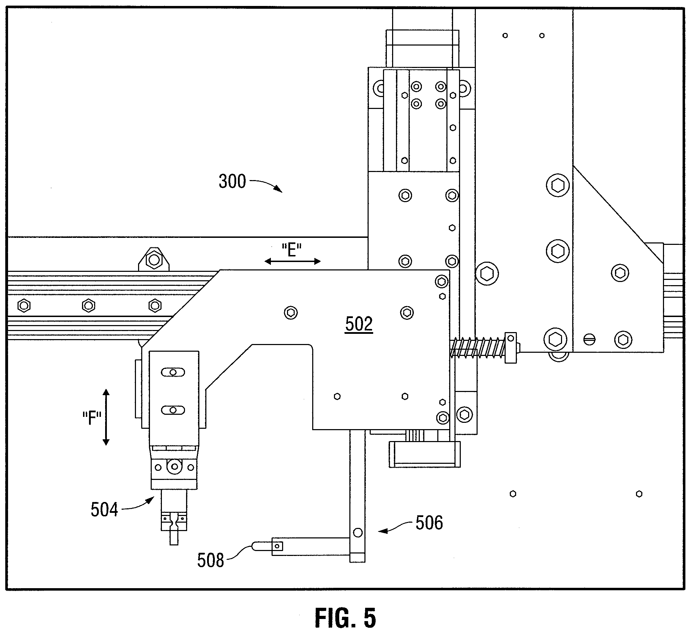

With reference still to FIG. 4, flipper gripping assembly 400 is configured to create loop 12 in thread 11. Gripping assembly 400 includes a rotating gripper 402 configured to selectively grasp a first end of thread 11. A mandrel 408 extends from rotating gripper 402 and includes a slot (not shown) configured to receive a hook 508 from carriage assembly 500 (FIG. 5). Rotating gripper 402 is configured to rotate relative to mandrel 408, in the direction of arrow "B", to loop thread 11 around mandrel 408. Flipper gripping assembly 400 is configured to move horizontally, in the direction of arrows "C", and vertically, in the direction of arrows "D".

With reference now to FIG. 5, carriage assembly 500 is configured to translate thread 11 through the loop forming process. Carriage assembly 500 includes a support member 502 having a tag end gripper 504 and a hook assembly 506. A hook 508 extends from hook assembly 506 and is configured to receive thread 11 thereabout. Carriage assembly 500 optionally includes a tensioning cylinder (not shown) for tensioning thread 11 with a predetermined force to test the strength of weld 15 (FIG. 1B). Carriage assembly 500 is configured to move horizontally, in the direction of arrows "E", and vertically, in the direction of arrows "F".

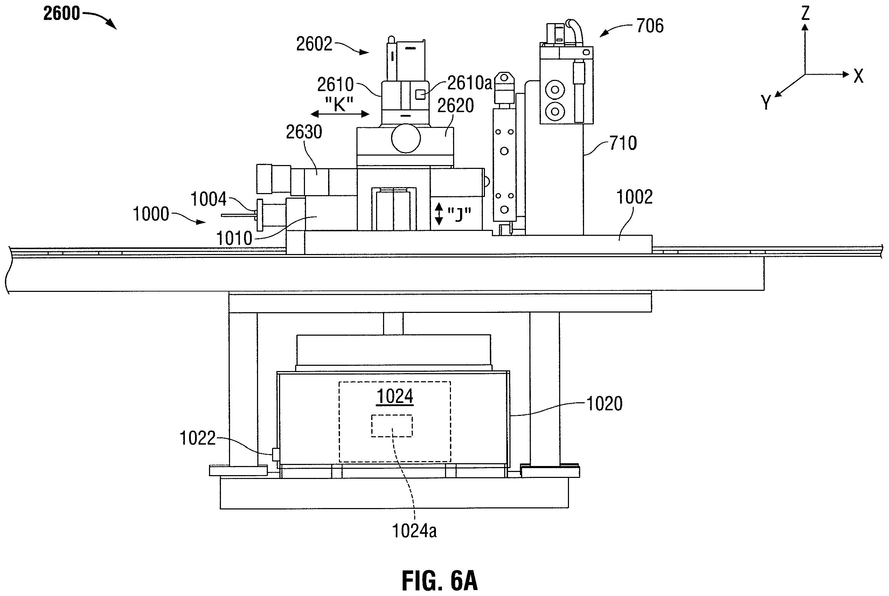

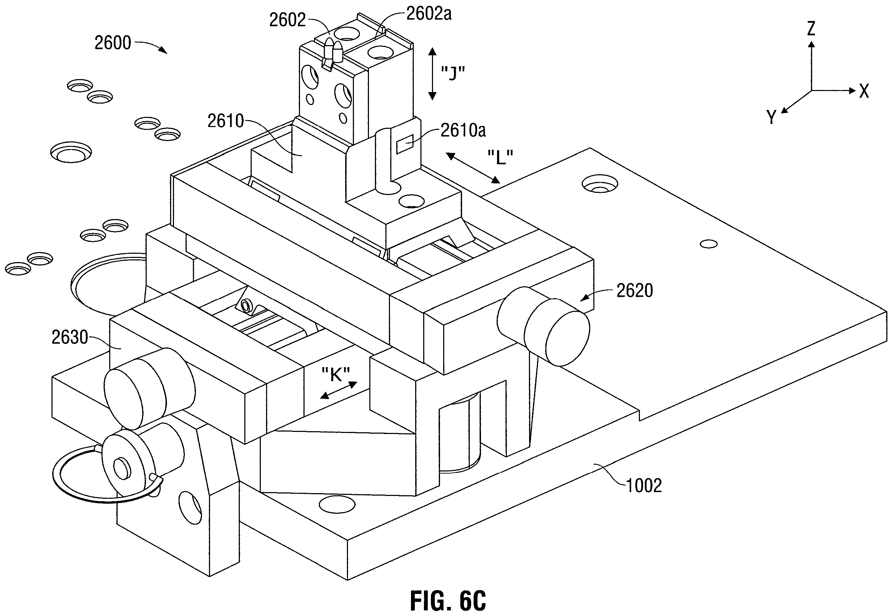

With reference to Figures 4 and 6A-6C, welding assembly 600 is configured to weld joined segment 15 in thread 11 to form loop 12 (FIG. 1A). Welding assembly 600 includes an active anvil assembly 2600 configured for selective engagement with an ultrasonic welding horn 604. In one embodiment, ultrasonic welding horn 604 may be manufactured by Branson Ultrasonics Corporation (Danbury, Ct). The term ultrasonic as used herein includes high frequency vibrations which are applied to workpieces being held together to create a solid state weld. Further, it should be understood that the term "welding horn" as used herein includes any component which transmits the mechanical vibrations (of a converted electrical signal) to the parts to be welded, e.g., a thread or suture. Active anvil assembly 2600 includes an anvil member 2602, a first sensor 2610a and a control assembly 1020.

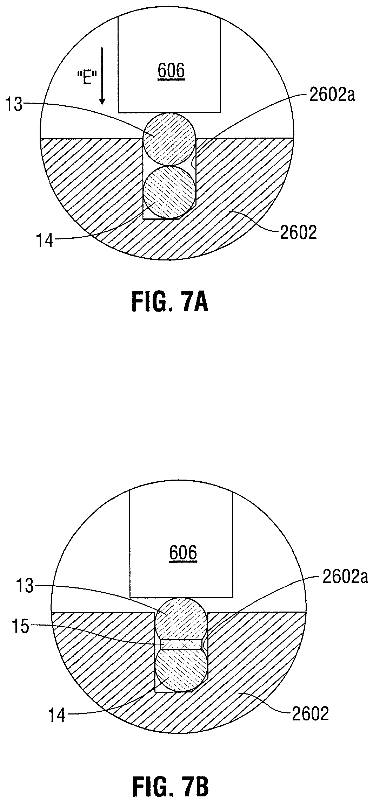

With reference to FIGS. 7A and 7B, ultrasonic welding horn 604 includes a flat die 606 configured to engage first section 13 of thread 11 during the welding step. As shown, anvil member 2602 defines a channel 2602a configured to receive the entire width of second section 14 and more than half the width of first section 13 of thread 11. In an alternative embodiment, flat die 606 may include a channel or recess (not shown) for receiving at least a portion of first section 13 of thread 11 instead of or in addition to the channel 2602a in the anvil member 2602.

Turning to FIGS. 6A-6C, the active anvil assembly 2600 will be described in greater detail. As described hereinabove, the active anvil assembly 2600 includes an anvil member 2602 and a first sensor 2610a which is operably connected to the anvil member 2602. The first sensor 2610a is incorporated within the anvil base 2610 and the first sensor 2610a interacts with and provides feedback to a control assembly 1020 (later described), effecting movement of the active anvil assembly 2600 during the loop forming process. First sensor 2610a is operably connected to the anvil member 2602 and may be configured to measure force, torque, distance and/or other conditions within active anvil assembly 2600. According to the invention, first sensor 2610a is configured to provide force feedback to active anvil assembly 2600. First sensor 2610a is disposed above first and second adjustable stages, 2620 and 2630, respectively. Either or both of anvil member 2602 and adjustable stages 2620, 2630, may be configured for movement in one or more directions prior to, during, or following the respective joining and trimming processes.

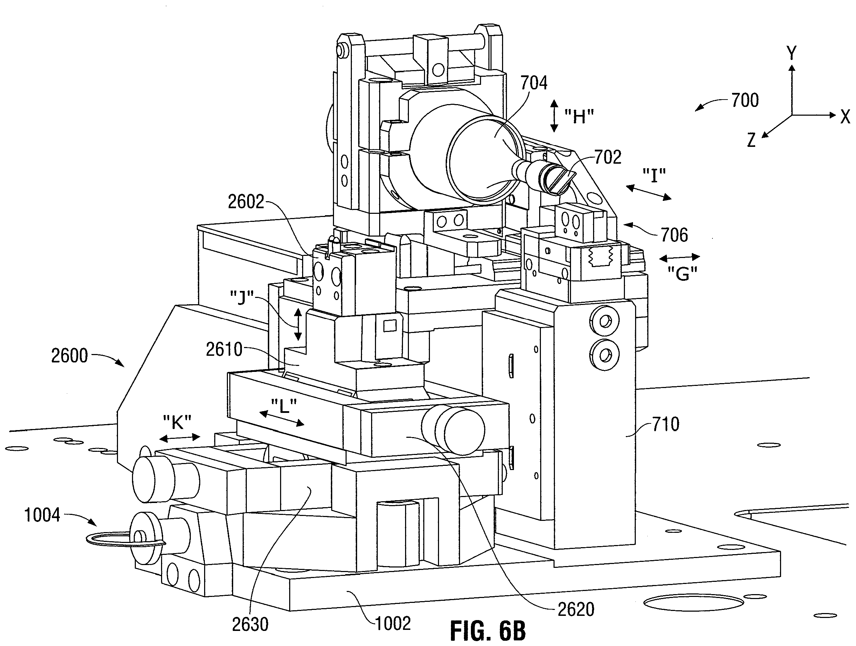

With particular reference to FIG. 6B, anvil base 2610 operably engages first adjustable stage 2620 and is configured to be selectively positioned relative thereto, as indicated by arrows "L." As shown in the drawings, first adjustable stage 2620 is configured to move the anvil base 2610 in a forward/backward or front/back direction. First adjustable stage 2620 operably engages second adjustable stage 2630 and is configured to be selectively positioned relative thereto, as indicated by arrows "K." As shown in drawings, second adjustable stage 2630 is configured to move anvil base 2610 in a lateral or side to side direction. Second adjustable stage 2630 operably engages a mounting member 1010 of mounting assembly 1000. It should be noted that although the adjustable stages 2620 and 2630 provided herein are described as part of the active anvil assembly 2600, it is envisioned, in an example not forming part of the invention, that the adjustable stages 2620 and 2630 may be provided as separate assemblies and/or may operate separately from anvil member 2602 and anvil base 2610.

With particular reference to FIG. 6A, the active anvil assembly 2600 further includes a mounting assembly 1000. Mounting assembly 1000 includes a mounting base 1002, mounting member 1010, and control assembly 1020. The mounting member 1010 is configured to further move the anvil member 2602. In particular, mounting member 1010 is operably engaged with mounting base 1002 and is configured to be raised and lowered relative to mounting base 1002, as indicated by arrows "J" to raise and lower anvil member 2602 relative to ultrasonic welding horn 604. As shown, mounting member 1010 is moved relative to mounting base 1002 by a control assembly 1020. A locking pin 1004 is configured for selective engagement with mounting member 1010. In particular, the locking pin 1004 is configured to prevent movement of mounting member 1010 relative to mounting base 1002.

Control assembly 1020 includes a motor 1024 that is configured to cause the raising and lowering, in other words, the vertical movement of mounting member 1010 relative to base 1002 and relative to the ultrasonic welding horn 604. Motor 1024 may include pneumatic or hydraulic cylinders, as shown, or any other mechanism suitable for selectively raising and/or lowering mounting member 1010 relative to base 1002. In one embodiment, motor 1024 includes a commercially available Allen Bradley servo motor. Control assembly 1020 may further include a second sensor 1024a for providing feedback, which may be used to position anvil member 2602 of active anvil assembly 2600 relative to ultrasonic welding horn 604 (FIG. 4). In particular, second sensor 1024a, may be configured to measure force, torque, distance, and/or other conditions experienced by active anvil assembly 2600. More specifically, second sensor 1024a is configured to provide torque and/or distance feedback to active anvil assembly 2600. Control assembly 1020 may also include one or more ports 1022 for selectively connecting control assembly 1020 with monitoring assembly 2000 (FIG. 2), a command station (not shown), a power source (not shown), and/or any other device or network (not shown).

With reference back to FIG. 6B, trimming assembly 700 is configured to cut tapered surface 17 of looped end portion 10. Trimming assembly 700 includes a trimming blade 702, which is powered by an ultrasonic horn 704. Trimming assembly 700 further includes a trim gripper 706 for gripping thread 11 as trimming blade 702 engages thread 11. Trim gripper 706 is configured to move laterally or side to side, in the direction of arrows "G," vertically or up and down, in the direction of arrows "H," and front and back, in the direction of arrow "I." Trim blade 702 is also configured to move laterally or side to side, vertically or up and down, and front and back, in the direction of arrows "G," "H," and "I." The speed, path, and frequency at which ultrasonic horn 704 and/or trimming blade 702 move, is adjustable to affect the configuration of tapered surface 17. Further, trim blade 702 is configured to be advanced and retracted relative to trim gripper 706. In one embodiment, trimming blade 702 is configured to be rotated one-hundred and eighty degrees (180°) about its longitudinal axis such that both cutting surfaces thereof may be used. Although shown adapted for use as an ultrasonic cutter, trimming assembly 700 may be configured cut tapered surface 17 without the use of ultrasonic energy in an example not forming part of the invention. In an alternate example, a laser may be used to cut tapered surface 17. Alternatively, trimming blade 702 may be heated to assist in the cutting of thread 11.

With reference still to FIG. 6B, trimming assembly 700 further includes a trimming base 710 securely connected with base 1002 of mounting assembly 1000. Base assembly 710 may be operably connected to control assembly 1022, monitoring assembly 2000 (FIG. 2), a command station (not shown), a power source (not shown), and/or any other device or network (not shown).

With reference back to FIG. 4, cutter assembly 800 is configured to cut thread 11 upon completion of the looped end forming process. Cutter assembly 800 includes a cutting blade 802. Cutter assembly 800 is configured to move parallel to initial gripper assembly 300, in the direction of arrows "A". Cutter assembly 800 is configured to cut thread 11 once thread 11 has attained an appropriate length. Cutter assembly 800 may be configured to cut a straight or tapered end on a proximal end 10a (FIG. 1A) of suture 10.

With reference to FIGS. 2 and 4, lengthening assembly 900 is configured to increase the length of thread 11 prior to thread 11 being cut by cutter assembly 800. As shown, lengthening assembly 900 includes a set of fixed rollers 902 and a set of adjustable rollers 904. Although shown including three and two rollers, respectively, sets of fixed and adjustable rollers 902, 904 may include any number of rollers. When thread 11 is received between fixed and adjustable rollers 902, 904, movement of adjustable rollers 904 relative to fixed rollers 902, in the direction of arrows "I", causes thread 11 to lengthen. The greater the number of rollers 902, 904, the less relative movement between rollers 902, 904 is necessary to lengthen thread 11.

Monitoring assembly 2000 is configured to monitor the various steps of the looped end forming process. Monitoring assembly 2000 includes a screen 2002 and a control panel 2004.

The operation of forming station 100 will now be described with reference to 2-6B. Thread 11 extends from spool 202 through first guide member 203 before being received about roller assembly 204. Thread 11 is wrapped around fixed rollers 204a and adjustable rollers 204b of roller assembly 204 four times, and is then received about first and second rollers 205, 206 before being received through second guide member 207. The number of times thread 11 is wrapped around each rollers 204a, 206 may vary depending on the size and/or composition of thread 11.

With continued reference to FIGS. 2-6B, thread 11 extends through second guide member 207 where it is grasped by initial gripper 302 prior to being grasped by rotating gripper 402. Initial grippers 302 then releases thread 11 and flipper gripping assembly 400 translates towards anvil member 2602 of active anvil assembly 2600 as carriage assembly 500 translates towards anvil member 2602 from the opposite direction. Flipper gripping assembly 400 and carriage assembly 500 are configured such that as carriage assembly 500 nears flipper gripping assembly 400, hook 508 of hook assembly 506 is received in the slot (not shown) of mandrel 407. Once hook 508 is received within the slot, rotating gripper 402 rotates, in the direction of arrow "B", to loop thread 11 about mandrel 407. Flipper gripping assembly 400 and carriage assembly 500 then move to position first and second sections 13, 14 of thread 11 within channel 2602a of anvil member 2602. As carriage assembly 500 approximates away from flipper gripping assembly 400, hook 508 extends from within the slot formed in mandrel 407 with thread 11 received thereabout.

In one embodiment, once first and second sections 13, 14 of thread 11 are received with channel 2602a of anvil member 2602, ultrasonic horn 604 is activated and flat die 606 is approximated towards anvil member 2602, in the direction of arrow "E". Engagement of flat welding die 606 with first section 13 of thread 11 causes first and second sections 13, 14 to weld together to form joined segment 15 (FIG. 1B). Alternatively, ultrasonic horn 604 may be positioned relative to anvil member 2602 prior to activating ultrasonic horn 604.

As discussed above with reference to FIGS. 6A and 6B, anvil member 2602 may instead, or additionally, be raised/lowered (arrows "J"), moved laterally (arrows "K") and/or moved front/back (arrows "L"), relative to ultrasonic horn 604 to position anvil member 2602 relative ultrasonic horn 604. Ultrasonic horn 604 may be activated prior to, during or after positioning of anvil member 2602 relative to ultrasonic horn 604. The positioning of anvil member 2602 may be accomplished using any number of methods. In each of the below disclosed methods, movement of anvil member 2602 is determined by the compressive force acting on first and second sections 13, 14 of thread 11 and is not a function of the initial position of ultrasonic horn 604. Additionally, in each of methods, ultrasonic horn 604 is not activated until anvil member 2602 is stationary.

In a first method, anvil member 2602 is moved relative to ultrasonic horn 604 until second sensor 1024a in control assembly 1020 senses a predefined torque value, at which point, the movement of anvil member 2602 is stopped and ultrasonic horn 604 is activated. In a second method, movement of anvil member 2602 stops when a predefined torque value is achieved, and then anvil member 2602 is moved an additional user defined distance. In yet a third method, anvil member 2602 is moved until a force feedback provided by first sensor 2610a of active assembly 2600 achieves a predefined set point, at which point, the movement of anvil member 2602 is stopped and ultrasonic horn 604 is activated. In a fourth method, movement of anvil member 2602 stops when a predefined force value, as measured by first sensor is achieved, and then anvil member 2602 is moved an additional user defined distance. The torque and/or force values and/or the additional user defined distance may vary depending on the size and type of thread being used and/or to effect different weld characteristics.

It is envisioned that ultrasonic horn 604 and anvil member 2602 may be moved simultaneously and/or individually to cause the forming of joined segment 15 and/or to effect the characteristics of joined segment 15 (FIG. 1A). Subsequent joining of first and second sections 13, 14 of thread 11, either or both of anvil member 2602 and ultrasonic horn 604 are approximated away from one another.

Once anvil member 2602 and ultrasonic horn 604 have been repositioned such that anvil member 2602 is spaced from ultrasonic horn 604, tail end gripper 504 of carriage assembly 500 grips a tail end (distal end) of thread 11 and rotating gripper 402 releases thread 11. Carriage assembly 500 then moves to position welded first and second section 13, 14 of thread 11 within gripping anvil 706 of trimming assembly 700. Gripping anvil 706 maintains thread 11 as trimming blade 704 of ultrasonic horn 702 is moved to cut tapered surface 17 (FIG. 1A) into first section 13 of thread 11. Alternatively, and discussed above with reference to FIGS. 6A and 6B, during forming of cut tapered surface 17, gripping anvil 706 may be raised/lowered (arrows "H"), moved laterally (arrows "G"), and/or moved front/back (arrows "I"), relative to trimming blade 702, to affect the characteristics of cut tapered surface 17 (FIG. 1A). Gripping anvil 706 then releases thread 11 and carriage assembly 500 continues to translate away from supply assembly 200 to extend the length of thread 11. It is envisioned that ultrasonic horn 702 and gripping anvil 706 may be moved simultaneously and/or individually to form cut tapered surface 17. Cutting assembly 900 is then activated to cut thread 11. Prior to the cutting of thread 11, tension is applied to loop 12 (FIG. 1A) of thread 11 by the tensioning cylinder (not shown) located within carriage assembly 500 to test the strength of weld 15. Optionally, thread 11 may engage lengthening assembly 800 to extend the length of thread 11 prior to cutting.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope of the claims. For example, it is envisioned that system 100 may include more than one welding assembly 600 and/or trimming assembly 700 to produce more than one suture 10 per activation.

## Claims

1. An active anvil assembly (2600) for use in forming a looped suture formed from a thread, the active anvil assembly comprising:
an anvil member (2602);
a first sensor (261 0a) operably connected to the anvil member, wherein the first sensor is configured for a force feedback; and
a control assembly (1020),
further including a first adjustable stage (2620) configured to translate forwards and backwards relative to an ultrasonic welding horn (604); wherein the anvil member (2602) is configured to operate with the ultrasonic welding horn (604) to join a first length of the thread and a second length of the thread to form a loop in the thread; and further including a second adjustable stage (2630) configured to translate laterally relative to the ultrasonic horn, and
wherein the first sensor (2610a) is disposed above the first and second adjustable stages, respectively,
further comprising a trimming assembly (700) comprising a trimming blade (702), powered by an ultrasonic horn (704) and further comprising a trim gripper (706) configured for gripping thread (11) as the trimming blade (702) engages the thread (11), wherein said trim gripper (706) is configured to move laterally (G), vertically (H) and front and back (I) the trimming assembly being configured to cut a tapered surface of the suture and **characterised in that** the speed, path and frequency at which the ultrasound horn and or trimming blade move is adjustable to effect the configuration of the surface of the thread to provide a tapered cut.

2. The active anvil assembly (2600) of claim 1, wherein the loop is formed at a distal end of the thread.

3. The active anvil assembly (2600) of claim 1 or claim 2, wherein the control assembly includes a motor (1024) configured to move a mounting member (1010) in relation to the ultrasonic welding horn; preferably wherein the mounting member is configured to move the anvil member (2602).

4. The active anvil assembly (2600) of claim 3, wherein the control assembly (1020) is configured to move the mounting member (1010) in relation to a mounting base (1002).

5. The active anvil assembly (2600) of claim 2, wherein the anvil member (2602) is selectively movable in at least first and second directions relative to the ultrasonic welding horn (604); and/or wherein the anvil member (2602) is configured for approximation towards and away from the ultrasonic welding horn.

6. The active anvil assembly (2600) of any preceding claim, wherein the anvil member (2602) includes a channel (2602a) configured to selectively receive at least a portion of the first length of thread.

7. The active anvil assembly (2600) of any preceding claim, wherein the control assembly (1020) is configured to vertically move the anvil member (2602).

8. A system for forming a looped suture, the system comprising:
the active anvil assembly (2600) of any preceding claim.

9. The system of claim 9, further comprising one or more of:
a flipper gripping assembly (400) configured for creating the loop in the thread;
a carriage assembly (500) configured for advancing the thread through the loop forming process;
a cutter assembly (800) for severing the thread upon completion of the loop forming process;
a thread lengthening assembly (900)configured for extending the length of the thread; and
a monitoring assembly (2000) configured for monitoring the forming process.

10. The system of claim 8 or 9, comprising the ultrasonic welding horn.

11. A method of forming a looped suture, the method comprising:
providing the system of claim 8, the system further including a welding assembly (600), wherein the welding assembly includes an ultrasonic welding horn (604) and the active anvil assembly (2600);
receiving a first length of thread adjacent a second length of thread between the ultrasonic welding horn and the active anvil assembly;
approximating at least one of the anvil member of the active anvil assembly and the ultrasonic welding horn towards the other until a predefined forceis sensed by the first sensor, which is disposed in the active anvil assembly;
activating the ultrasonic welding horn;
joining the adjacent first and second lengths of thread; and
approximating at least one of the anvil member (2602) and the ultrasonic welding horn away from the other of the ultrasonic welding horn and anvil member,
operably engaging the joined first and second lengths of thread with the trim gripper (706) of the trimming assembly (700);
approximating the trim gripper towards the ultrasonic horn (704) of the trimming assembly (700);
removing excess thread from the joined first and second lengths of thread; and
approximating the trim gripper away from the ultrasonic horn of the trimming assembly.

## Patentansprüche

1. Aktive Ambossanordnung (2600) zur Verwendung beim Ausbilden einer schlaufenförmigen Naht, die aus einem Gewinde ausgebildet ist, die aktive Ambossanordnung umfassend:
ein Ambosselement (2602);
einen ersten Sensor (2610a), der mit dem Ambosselement betriebsfähig verbunden ist, wobei der erste Sensor für eine Kraftrückkopplung konfiguriert ist; und
eine Steueranordnung (1020),
ferner einschließlich einer ersten einstellbaren Stufe (2620), die konfiguriert ist, um relativ zu einem Ultraschallschweißhorn (604) vorwärts und rückwärts verschoben zu werden; wobei das Ambosselement (2602) konfiguriert ist, um mit dem Ultraschallschweißhorn (604) in Betrieb zu sein, um eine erste Länge des Gewindes und eine zweite Länge des Gewindes zu verknüpfen, um eine Schlaufe in dem Gewinde auszubilden;
und ferner einschließlich einer zweiten einstellbaren Stufe (2630), die konfiguriert ist, um sich relativ zu dem Ultraschallhorn seitlich zu verschieben, und
wobei der erste Sensor (2610a) über der ersten beziehungsweise der zweiten einstellbaren Stufe angeordnet ist,
ferner umfassend eine Trimmanordnung (700), umfassend eine Trimmklinge (702), die durch ein Ultraschallhorn (704) angetrieben wird und ferner umfassend einen Trimmgreifer (706), der für das Greifgewinde (11) konfiguriert ist, wenn die Trimmklinge (702) das Gewinde (11) in Eingriff nimmt, wobei der Trimmgreifer (706) konfiguriert ist, um sich seitlich (G), vertikal (H) und vor und zurück (I) zu bewegen, wobei die Trimmanordnung konfiguriert ist, um eine sich verjüngende Oberfläche der Naht zu schneiden, und
**dadurch gekennzeichnet, dass** die Geschwindigkeit, der Pfad und die Frequenz, mit der sich das Ultraschallhorn und/oder die Trimmklinge bewegen, einstellbar ist, um die Konfiguration der Oberfläche des Gewindes zu bewirken, um einen sich verjüngenden Schnitt bereitzustellen.

2. Aktive Ambossanordnung (2600) nach Anspruch 1,
wobei die Schlaufe an einem distalen Ende des Gewindes ausgebildet ist.

3. Aktive Ambossanordnung (2600) nach Anspruch 1 oder 2, wobei die Steueranordnung einen Motor (1024) einschließt, der konfiguriert ist, um ein Montageelement (1010) in Bezug auf das Ultraschallschweißhorn zu bewegen; vorzugsweise wobei das Montageelement konfiguriert ist, um das Ambosselement (2602) zu bewegen.

4. Aktive Ambossanordnung (2600) nach Anspruch 3, wobei die Steueranordnung (1020) konfiguriert ist, um das Montageelement (1010) in Bezug auf eine Montagebasis (1002) zu bewegen.

5. Aktive Ambossanordnung (2600) nach Anspruch 2, wobei das Ambosselement (2602) in mindestens einer ersten und einer zweiten Richtung relativ zu dem Ultraschallschweißhorn (604) selektiv bewegbar ist; und/oder wobei das Ambosselement (2602) für eine Annäherung zu dem Ultraschallschweißhorn hin und davon weg konfiguriert ist.

6. Aktive Ambossanordnung (2600) nach einem der vorstehenden Ansprüche, wobei das Ambosselement (2602) einen Kanal (2602a) einschließt, der konfiguriert ist, um mindestens einen Abschnitt der ersten Gewindelänge selektiv aufzunehmen.

7. Aktive Ambossanordnung (2600) nach einem der vorstehenden Ansprüche, wobei die Steueranordnung (1020) konfiguriert ist, um das Ambosselement (2602) vertikal zu bewegen.

8. System zum Ausbilden einer schlaufenförmigen Naht, das System umfassend:
die aktive Ambossanordnung (2600) nach einem der vorstehenden Ansprüche.

9. System nach Anspruch 9, ferner umfassend eines oder mehrere von:
einer Flipper-Greifanordnung (400), die zum Erzeugen der Schlaufe in dem Gewinde konfiguriert ist;
einer Schlittenanordnung (500), die zum Vorschieben des Gewindes durch den Schlaufenausbildungsprozess konfiguriert ist;
einer Schneideanordnung (800) zum Durchtrennen des Gewindes nach Abschluss des Schlaufenausbildungsprozesses;
einer Gewindeverlängerungsanordnung (900), die zum Erstrecken der Länge des Gewindes konfiguriert ist; und
einer Überwachungsanordnung (2000), die zum Überwachen des Ausbildungsprozesses konfiguriert ist.

10. System nach Anspruch 8 oder 9, umfassend das Ultraschallschweißhorn.

11. Verfahren zum Ausbilden einer schlaufenförmigen Naht, das Verfahren umfassend:
Bereitstellen des Systems nach Anspruch 8, wobei das System ferner eine Schweißanordnung (600) einschließt, wobei die Schweißanordnung ein Ultraschallschweißhorn (604) und die aktive Ambossanordnung (2600) einschließt,
Aufnehmen einer ersten Gewindelänge angrenzend an eine zweite Gewindelänge zwischen dem Ultraschallschweißhorn und der aktiven Ambossanordnung;
Annähern mindestens eines des Ambosselements der aktiven Ambossanordnung und des Ultraschallschweißhorns zu dem anderen hin, bis eine vordefinierte Kraft durch den ersten Sensor erfasst wird, der in der aktiven Ambossanordnung eingerichtet ist;
Aktivieren des Ultraschallschweißhorns;
Verknüpfen der angrenzenden ersten und zweiten Gewindelänge; und
Annähern mindestens eines des Ambosselements (2602) und des Ultraschallschweißhorns von dem anderen des Ultraschallschweißhorns und des Ambosselements weg,
betriebsfähiges Ineingriffnehmen der verknüpften ersten und zweiten Gewindelänge mit dem Trimmgreifer (706) der Trimmanordnung (700);
Annähern des Trimmgreifers zu dem Ultraschallhorn (704) der Trimmanordnung (700) hin;
Entfernen von überschüssigem Gewinde aus der verknüpften ersten und zweiten Gewindelänge; und
Annähern des Trimmgreifers von dem Ultraschallhorn der Trimmanordnung weg.

## Revendications

1. Ensemble enclume actif (2600) destiné à être utilisé pour former une suture en boucle formée à partir d'un fil, l'ensemble enclume actif comprenant :
un élément enclume (2602) ;
un premier capteur (2610a) relié de manière fonctionnelle à l'élément d'enclume, le premier capteur étant configuré pour une rétroaction de force ; et
un ensemble de commande (1020),
comportant en outre un premier étage réglable (2620) conçu pour se déplacer en translation vers l'avant et vers l'arrière par rapport à un pavillon de soudage ultrasonique (604) ; l'élément d'enclume (2602) étant conçu pour fonctionner avec le pavillon de soudage ultrasonique (604) afin de rejoindre une première longueur du fil et une seconde longueur du fil pour former une boucle dans le fil ; et comportant en outre un second étage réglable (2630) conçu pour se déplacer en translation latéralement par rapport au pavillon ultrasonique, et
le premier capteur (2610a) étant disposé au-dessus des premier et second étages réglables, respectivement,
comprenant en outre un ensemble de coupe (700) comprenant une lame de coupe (702), actionnée par un pavillon ultrasonique (704) et comprenant en outre un dispositif de préhension de coupe (706) configuré pour saisir le fil (11) lorsque la lame de coupe (702) vient en prise avec le fil (11), ledit dispositif de préhension de coupe (706) étant configuré pour se déplacer latéralement (G), verticalement (H) et en avant et en arrière (I), l'ensemble de coupe étant conçu pour couper une surface effilée de la suture et **caractérisé en ce que** la vitesse, la trajectoire et la fréquence, auxquelles se déplacent le pavillon ultrasonique et ou la lame de coupe, sont réglables pour effectuer la configuration de la surface du fil afin de fournir une coupe effilée.

2. Ensemble enclume actif (2600) selon la revendication 1,
dans lequel la boucle est formée à une extrémité distale du fil.

3. Ensemble enclume actif (2600) selon la revendication 1 ou la revendication 2, dans lequel l'ensemble de commande comporte un moteur (1024) configuré pour déplacer un élément de montage (1010) par rapport au pavillon sonore de soudage ultrasonique ; de préférence, l'élément de montage étant conçu pour déplacer l'élément d'enclume (2602).

4. Ensemble enclume actif (2600) selon la revendication 3, dans lequel l'ensemble de commande (1020) est configuré pour déplacer l'élément de montage (1010) par rapport à une base de montage (1002).

5. Ensemble enclume actif (2600) selon la revendication 2, dans lequel l'élément d'enclume (2602) est sélectivement mobile dans au moins des première et seconde directions par rapport au pavillon de soudage ultrasonique (604) ; et/ou l'élément d'enclume (2602) étant conçu pour se rapprocher et s'éloigner du pavillon de soudage ultrasonique.

6. Ensemble enclume actif (2600) selon l'une quelconque revendication précédente, dans lequel l'élément d'enclume (2602) comporte un canal (2602a) conçu pour recevoir sélectivement au moins une partie de la première longueur de fil.

7. Ensemble enclume actif (2600) selon l'une quelconque revendication précédente, dans lequel l'ensemble de commande (1020) est configuré pour déplacer verticalement l'élément d'enclume (2602).

8. Système de formation d'une suture en boucle, le système comprenant :
l'ensemble enclume actif (2600) selon l'une quelconque des revendications précédentes.

9. Système selon la revendication 9, comprenant en outre un ou plusieurs parmi :
un ensemble de préhension de déflecteur (400) conçu pour créer la boucle dans le filetage ;
un ensemble chariot (500) conçu pour faire avancer le fil à travers le processus de formation de boucle ;
un ensemble de coupe (800) pour couper le fil à l'issue du processus de formation de boucle ;
un ensemble allongement de fil (900) conçu pour étendre la longueur du filetage ; et
un ensemble de surveillance (2000) configuré pour surveiller le processus de formation.

10. Système selon la revendication 8 ou 9, comprenant le pavillon de soudage ultrasonique.

11. Procédé de formation d'une suture en boucle, le procédé comprenant :
la fourniture du système selon la revendication 8, le système comportant en outre un ensemble de soudage (600), l'ensemble de soudage comportant un pavillon de soudage ultrasonique (604) et l'ensemble enclume actif (2600) ;
la réception d'une première longueur de fil adjacente à une seconde longueur de fil entre le pavillon de soudage ultrasonique et l'ensemble enclume actif ;
l'approximation de l'élément d'enclume de l'ensemble enclume actif et du pavillon de soudage ultrasonique vers l'autre jusqu'à ce qu'une force prédéfinie soit détectée par le premier capteur, qui est disposé dans l'ensemble enclume actif ;
l'activation du pavillon de soudage ultrasonique ;
l'assemblage des première et seconde longueurs de fil adjacentes ; et
l'approximation de l'élément d'enclume (2602) et/ou du pavillon de soudage ultrasonique à l'écart de l'autre du pavillon de soudage ultrasonique et de l'élément d'enclume,
la mise en prise fonctionnelle des première et seconde longueurs de fil jointes avec le dispositif de préhension de coupe (706) de l'ensemble de coupe (700) ;
l'approximation du dispositif de préhension de coupe vers le pavillon ultrasonique (704) de l'ensemble de coupe (700) ;
le retrait de l'excès de fil des première et seconde longueurs de fil jointes ; et
l'approximation du dispositif de préhension de coupe à l'écart du pavillon ultrasonique de l'ensemble de coupe.
